Europäisches Patentamt

European Patent Office

Office européen des brevets

(19)

(11) Publication number: **0 061 242**
A1

## (12) EUROPEAN PATENT APPLICATION

(21) Application number: **82301047.5**

(22) Date of filing: **02.03.82**

(51) Int. Cl.³: **C 08 L 81/04**
**B 01 J 31/26, A 61 K 6/10**

(30) Priority: **25.03.81 US 247231**

(43) Date of publication of application:
**29.09.82 Bulletin 82/39**

(84) Designated Contracting States:
**AT BE CH DE FR GB IT LI NL SE**

(71) Applicant: SYBRON CORPORATION
1100 Midtown Tower
Rochester, NY 14604(US)

(72) Inventor: Larson, Melvin L.
2309 Georgetown Boulevard
Ann Arbor Michigan 48105(US)

(74) Representative: Oliver, Roy Edward et al,
POLLAK,MERCER & TENCH High Holborn House 52-54
High Holborn
London WC1V 6RY(GB)

(54) Catalyst compositions for curing polythiopolymercaptan polymers.

(57) A catalyst composition, for mouth-temperature curing of liquid polythiopolymercaptan polymers to produce elastomeric dental impression materials, comprises acid-treated submicron zinc oxide, the treating acid preferably being propionic acid, and purified benzothiazyl disulphide (2,2'-dithiobis-(benzothiazole). The composition contains at least 20% by weight of the acid-treated submicron zinc oxide, not more than 20% and preferably 5% to 20% by weight of substantially pure benzothiazyl disulphide and 20% to 50% by weight of plasticizer.

- 1 -

CATALYST COMPOSITIONS FOR CURING POLYTHIOPOLYMERCAPTAN POLYMERS

DESCRIPTION

The present invention relates to dental compositions and is more particularly concerned with catalyst compositions which are suitable for use in curing base paste compositions, especially in forming dental impressions.

Although there is substantial patent literature dealing with room temperature curing of polythiopolymercaptan polymers, primarily for sealant, coating and adhesive applications, there is very little in the prior art relating to the fast curing of such polymers for use as dental impression materials.

The main requirements of dental impression materials are that they undergo curing inside the mouth within about 10 minutes, give certain properties of compression set, exhibit resistance to stress and have dimensional stability. Only materials containing zinc oxide and giving relatively rapid curing are discussed below as being relevant to the novelty and superiority of impression materials based upon use of the catalyst compositions of the present invention.

It has long been recognized that zinc oxide by itself will promote slow room-temperature curing of polysulphide polymers containing thiol groups. The problem has been to obtain a catalyst composition which gives accelerated curing of compositions based upon such polymers and so makes them sufficiently fast and appropriate for dental impression use. US-PS 3,362,927 discloses the addition of dimethyl sulphoxide to a base paste containing a polythiopolymercaptan polymer, zinc oxide being present in the catalyst. However, dimethyl sulphoxide has independent and undesirable oxidizing properties towards thiols, producing a dimethyl sulphide by-product. Activation of zinc oxide by means of aqueous acetic acid is disclosed in US-PS 3,637,612, but the presence of water is undesirable, because it leads to shrinkage of the resultant elastomer.

US-PS 3,923,754 discloses the use of at least 20 parts by weight of benzothiazyl disulphide, based upon 100 parts by weight of the polythiopolymercaptan polymer, in combination with zinc oxide. As discussed in more detail below, the present invention successfully uses purified benzothiazyl disulphide in lower concentrations in combination i.a. with submicron zinc oxide activated by treatment with propionic acid.

According to the present invention, a catalyst composition is provided, which is suitable for use in converting a paste containing a polythiopolymercaptan polymer into a dental impression material and can be used in a time of less than about 10 minutes, which comprises a paste containing zinc oxide and benzothiazyl disulphide, characterised in that the paste comprises, by weight, at least 20% of organic-acid-treated submicron size zinc oxide, 5% to 20% of substantially pure benzothiazyl disulphide and 20% to 50% of plasticizer.

When a catalyst composition of the invention is used with a polythiopolymercaptan polymer in dental work, the resulting elastomeric impression has the properties of low compression set, resistance to stress, dimensional stability and low toxicity suitable for making dental appliances by methods well known in the art.

An example of a liquid polythiopolymercaptan polymer which is transformed into a suitable elastomer by use of a catalyst composition of this invention is sold under the tradename "Thiokol LP-2" and is manufactured by the Chemical Division of Thiokol Corporation. This polymer has the following properties:

| | |
|---|---|
| Viscosity: | 470 poises at $25^{O}C$ ($77^{O}F$) |
| Molecular weight: | 4000 (average) |
| Mercaptan content: | 1.5% - 2.0% by weight |
| Crosslinking: | 2.0 mole percent due to 1,2,3-trichloropropane reaction |

Although the average structure of "Thiokol LP-2" is $HS(C_2H_4OCH_2OC_2H_4SS)_{23}C_2H_4OCH_2OC_2H_4SH$, indicating terminal thiol groups, pendant cross-linking thiol groups are also present to assist in the development of elastomeric properties. This polymer is the essential component of the base paste of the normal two-paste system (base plus catalyst), where mixing occurs just prior to mouth placement.

The conventional catalyst used for curing "Thiokol LP-2" contains substantial amounts of lead peroxide, which can be objectionable because of its toxicity and its dark-brown colour which causes staining of skin and clothing and is especially difficult to remove.

The catalyst compositions of the present invention, in which propionic-acid-treated submicron zinc oxide is preferably used, contain purified benzothiazyl disulphide (MBTS). The orally toxic dose (LD50) of MBTS for the rat is quite high (7 g/kg), thus greatly exceeding the limiting dose (5 g/kg) distinguishing human lethality, which is quite high.

The compositions of the present invention also eliminate the objectionable colour and stain associated with prior art compositions, since all the components are essentially light in colour. Therefore, the catalyst paste can be coloured with any appropriate pigment (chemically inert and non-toxic), for instance to provide colour contrast as an indication of thorough mixing with the base paste.

An additional advantage of the present invention is the absence of significant shrinkage of the resultant elastomeric impression, since the polymer catalyst system does not involve any oxidative condensation reaction of the thiols giving water as a by-product. Metal peroxide curatives, e.g. lead peroxide, zinc peroxide or calcium peroxide, and cupric hydroxide cause curing by such condensation reactions, so that the resultant impressions exhibit significant negative dimensional change (shrinkage). Organic hydroperoxide curatives also give significant shrinkage.

By comparison, the compositions of the invention, typically based on propionic-acid-treated zinc oxide and purified benzothiazyl disulphide, do not involve an oxidative attack on the thiol groups at the temperature of the mouth, i.e. less than $37^{\circ}C$ ($98.6^{\circ}F$). Therefore, no volatile water by-product is formed, resulting in elastomeric impressions having considerable dimensional stability.

In using the catalyst compositions of the present invention, preferably below 20 parts per hundred are employed by weight of the liquid polythiopolymercaptan polymer, e.g. "Thiokol LP-2". Although a paste containing propionic-acid-activated zinc oxide slowly cures the base paste containing the "Thiokol LP-2", the interaction with MBTS enables the catalyst to cause rapid curing of elastomers after mouth placement for less than about 10 minutes. In some instances, this interaction may be promoted thermally by heat-treating the catalyst paste before it is mixed with the base paste.

Since MBTS used alone causes rubber reversion of cured "Thiokol LP-2", the presence of an excess of the zinc oxide in the catalyst composition is required. Therefore, the compositions of the invention preferably have a ZnO:MBTS weight ratio greater than 2 to 1. In addition, reversion of the elastomer to a less-cured state is promoted by organic base impurities found in substantial amounts, e.g. 5% - 15%, in normal commercial-grade MBTS. This is because MBTS is manufactured by the oxidation of crude 2-mercaptobenzothiazole (MBT) prepared by the autoclave reaction of aniline, sulphur and carbon disulphide. This crude MBT contains a variety of impurities in substantial amounts, 5% - 15% of which can remain in normal commercial-grade MBTS. This crude MBT contains various impurities, such as benzothiazole, anilinobenzothiazole, 3-(2-benzothiazolyl)-2-benzothiazolinethione, phenyl isothiocyanate, diphenylthiourea and 2-aminothiophenol, as well as unreacted aniline and sulphur (see US-PS 3,904,638). The organic impurities are believed to be deleterious, in that they promote rubber reversion subsequent to the cross-linking, apparently by activation of MBTS. Therefore, the MBTS used in carrying out the present invention requires purification e.g. by crystallization or efficient solvent extraction, in order to remove these reversion-inducing impurities. If these impurities are not sufficiently removed, they reduce the working time of the mixed paste below the necessary 2-3 minutes during room-temperature storage. Such MBTS-containing catalysts require employment at lower concentrations with the base and prior heat-treatment to achieve near stable activity (see Example IV).

The catalyst compositions of the present invention involve activation of the thiol group of the polymer, e.g. "Thiokol LP-2", by coordination with zinc-propionate-coated submicron size zinc oxide, which in combination with MBTS gives the rapid crosslinking reaction required for elastomeric cure. Using substantially pure MBTS lowers the amount required to less than 20 parts per hundred parts of LP-2 by weight, thereby diminishing its rubber reversion influence. The excess propionic-acid-treated zinc oxide has the function of displacing the reaction towards crosslinking and inhibiting the reversion influence of MBTS. The catalyst paste heat-activation requirement and its mix ratio with the base paste depend upon the MBTS purity.

The catalyst paste composition of this invention preferably comprises amounts in the stated ranges of the zinc oxide curative, the benzothiazyl disulphide (MBTS) and the inert plasticizer, together with inert pigment, inert flavourant and inert filler, if desirable. The concentrations of the various ingredients, in weight percent, can be as follows:

| | |
|---|---|
| organic-acid-treated zinc oxide | 40% - 60% |
| substantially pure benzothiazyl disulphide | 5% - 20% |
| plasticizer | 20% - 50% |
| colouring pigment | 0.1% - 2.5% |
| flavourant | 0.1% - 2.5% |
| filler | up to 35% |

The zinc oxide preferably contains 90% - 100% of particles less than 1 micron ($10^{-3}$ mm) in spherical diameter and should be treated with propionic acid or some other organic acid, preferably carboxylic, to provide a zinc carboxylate or other coating. The preferred propionic-acid-treated zinc oxide used in carrying out this invention contains zinc propionate and/or propionic acid equivalent to 0.2% - 0.7% by weight of propionic acid. Depending upon the efficacy of the propionic acid treatment, the MBTS activity and the base:catalyst mix ratio, this zinc oxide can be employed in the catalyst paste to the extent of 20-100 parts by weight per 100 parts by weight of the polythiopolymercaptan polymer.

The MBTS purity is defined essentially by catalyst performance with respect to promotion of elastomeric curing versus excessive acceleration-reversion. In terms of MBTS-MBT content, it normally has a minimum MBTS purity of 92% and a maximum MBT content of 5% following extraction purification. Crystallisation purification, of course, increases this MBTS purity. The purified MBTS can be employed in the catalyst paste in an amount below 20 parts per 100 and, normally, 10-20 parts per 100 parts by weight of the polythiopolymercaptan liquid polymer.

As is to be expected with virtually any chemical production process, batch to batch variations occur among lots of benzothiazyl disulphide produced by a given source, as well as variations between sources. Not only do these variations appear in terms of MBTS and MBT contents, but also as impurity contents which are as-yet unidentified. Thus, even after extensive purification, either by solvent extraction or by recrystallization, two batches of benzothiazyl disulphide of very similar MBTS and MBT contents can have quite dissimilar catalytic activities in promoting the low-temperature vulcanization of polythiopolymercaptan pre-polymers by propionic-acid-treated zinc oxide.

This apparent dilemma is controllable in carrying out the present invention by "baby batch" testing of the reactivity of the various batches of benzothiazyl disulphide, followed by judicious formulation and blending, to obtain the desired degree of reactivity and suitable physical properties in order to meet adequately the requirements of the invention.

Suitable inert fillers which may be used with this catalyst are titanium dioxide and aluminium oxide. Phthalate esters, such as dibutyl phthalate, are suitable inert plasticizers. Other suitable kinds of plasticizer are diphenyl alkyl phosphate esters, polyalkylene glycol carboxylates and N-alkyl-substituted toluene sulphonamides. The catalyst paste is also suitably coloured with inert pigments, such as copper phthalocyanine blue or chlorinated copper phthalocyanine green, and advantageously contains appropriate flavourants, such as 1-carvone or synthetic peppermint oil.

As stated above, some catalyst pastes are formulated so as to require heat-treatment activation. The base paste associated with the catalyst paste of this invention preferably contains the polythiopolymercaptan liquid polymer having crosslinking thiol groups, fillers and a plasticizer. The polymer available under the trade name "Thiokol LP-2" has an average molecular weight of 4000, a viscosity of about 470 poises at 25°C and 2 mole percent of crosslinking functionality, based on reacted 1,2,3-trichloropropane. Another suitable polymer is available under the tradename "Thiokol LP-32" which has 0.5 mole percent of crosslinking functionality.

The catalyst paste of this invention and the base paste are mixed together in appropriate proportions to obtain a cured elastomeric dental impression after mouth placement for less than about 10 minutes. The resultant impression has suitable physical properties for processing the desired dental restorative prothesis. These properties include the special advantage of low shrinkage.

The following examples are illustrative of the properties obtained from catalyst paste compositions of this invention. In these examples, the zinc oxide and the benzothiazyl disulphide are those heretofore described in detail. Each solid component of the catalyst paste was blended by mixing with dibutyl phthalate. The catalyst paste was hand-mixed for 1 minute in an equi-weight combination, in the case of Examples I and II, and in ratios of 3:2 for Example III and 3:1 for Example IV, with the base paste, which is sold under the tradename "Permlastic" by Kerr Manufacturing Co., a Division of Sybron Corporation. The catalyst and the base paste are mixed together by spatulation. The catalyst compositions of the examples are based upon the previously-designated combinations containing 100 parts by weight of "Thiokol LP-2". "Working time" is defined by the American Dental Association Specification No. 19 for elastomeric impression materials (American National Standard Z156.19-1971). The compression set, strain in compression and dimensional stability are defined by Revised American Dental Association Specification No. 19 (J. Am. Dental Assoc. 1977, 94,733-741).

EXAMPLE I

| Ingredients | Parts by Weight |
|---|---|
| "Thiokol LP-2" in base paste | 100.00 |
| Catalyst paste: | |
| Zinc oxide | 82.38 |
| MBTS | 13.56 |
| Dibutyl phthalate | 39.15 |
| Titanium dioxide | 32.88 |
| Pigment (copper phthalocyanine blue) | 1.53 |

Working time                    3-4 minutes

A 6-minute cure at $32^{O}C$ gave the following physical properties:

Compression set          1.4%

Strain in Compression          9.1%

Two weeks later, the room-temperature rubber specimens were found to have strain in compression of 9.8%, demonstrating only slight reversion.

## EXAMPLE II

| Ingredients | Parts by Weight |
|---|---|
| "Thiokol LP-2" in base paste | 100.00 |
| Catalyst paste: | |
| Zinc oxide | 67.80 |
| MBTS | 12.03 |
| Dibutyl phthalate | 44.75 |
| Titanium dioxide | 43.39 |
| Pigment | 1.53 |
| Working time | 4-5 minutes |

A 6-minute cure at 32°C gave the following physical properties:

| | |
|---|---|
| Compression set | 2.7% |
| Strain in Compression | 11.3% |

Two weeks later, the room-temperature stored specimens were found to have strain in compression of 8.9%. The dimensional change after 24 hours at room temperature was from -0.1% to -0.2%.

## EXAMPLE III

| Ingredients | Parts by Weight |
|---|---|
| "Thiokol LP-2" in base paste | 100.00 |
| Catalyst paste: | |
| Zinc oxide | 47.16 |
| MBTS | 16.02 |
| Dibutyl phthalate | 24.92 |
| Pigment | 0.89 |
| Working time | 7-8 minutes |

A 6-minute cure at 32°C gave the following physical properties:

| | |
|---|---|
| Compression set | 1.3% |
| Strain in Compression | 15.1% |

Nine days later, this strain in compression had increased to 17.7%, demonstrating only slight reversion.

EXAMPLE IV

| Ingredients | Parts by Weight |
|---|---|
| "Thiokol LP-2" in base paste | 100.00 |
| Catalyst paste: | |
|     Zinc oxide | 26.28 |
|     MBTS | 10.66 |
|     Dibutyl phthalate | 16.07 |
|     Pigment | 0.50 |
|     Flavourant | 2.01 |

The catalyst paste was given a 9-day heat-treatment at $60^{\circ}$C.

| | |
|---|---|
| Working time | 6-7 minutes |

An 8-minute cure at $32^{\circ}$C gave the following physical properties:

| | |
|---|---|
| Compression set | 2.3% |
| Strain in compression | 9.6% |

Nine days later, this strain in compression was 8.3%.

CLAIMS:

1. A catalyst composition which comprises a paste containing zinc oxide and benzothiazyl disulphide, characterised in that the paste comprises, by weight, at least 20% of organic-acid-treated submicron size zinc oxide, 5% to 20% of substantially pure benzothiazyl disulphide and 20% to 50% of plasticiser.

2. A catalyst composition according to claim 1, which includes, by weight, 0.1% to 2.5% of a colouring pigment and 0.1% to 2.5% of a flavourant.

3. A catalyst composition according to claim 1 or 2, which includes, by weight, up to 35% of at least one filler.

4. A catalyst composition according to any preceding claim, in which the zinc oxide is of submicron size in particle diameter, 90% being less than 1 micron $(10^{-3}$ mm) in diameter, and has been treated with propionic acid so as to contain 0.2% to 0.7% by weight of zinc propionate.

5. A catalyst composition according to any preceding claim, in which the benzothiazyl disulphide has a purity of at least 92% by weight.

6. A composition according to any preceding claim, in which the zinc oxide and the benzothiazyl disulphide are present in a weight ratio of at least 2 to 1.

7. A catalyst composition according to any preceding claim, which contains 40% to 60% by weight of the zinc oxide.

## European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | US-A-3 923 754 (MICHAEL A.PELLICO) *Claims; examples* | 1 | C 08 L 81/04 B 01 J 31/26 A 61 K 6/10 |
| D,Y | US-A-3 637 612 (EUGENE R.BERTOZZI) *Claims* | 1 | |

**TECHNICAL FIELDS SEARCHED (Int. Cl. 3)**

C 08 L

The present search report has been drawn up for all claims

| Place of search THE HAGUE | Date of completion of the search 24-06-1982 | Examiner MALHERBE Y.J.M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82